# EUROPEAN PATENT SPECIFICATION

(11) **EP 4 497 302 B1**
(45) Date of publication and mention of the grant of the patent: **14.10.2026**
(21) Application number: 23715437.2
(22) Date of filing: 23.03.2023
(51) Int. Cl.: H04W 88/08, A61B 5/00, H04L 67/12, H04L 69/08, H04W 84/12, H04W 88/10, H04W 84/10

(54) **METHOD AND SYSTEM FOR PRIVATE PATIENT MONITORING NETWORKS**
VERFAHREN UND SYSTEM FÜR PRIVATE PATIENTENÜBERWACHUNGSNETZWERKE
PROCÉDÉ ET SYSTÈME POUR LES RÉSEAUX PRIVÉS DE SURVEILLANCE DES PATIENTS

(30) Priority: 23.03.2022 US 202263322675 P
(43) Date of publication of application: 29.01.2025
(73) Proprietor: Koninklijke Philips N.V., 5656 AE Eindhoven (NL)
(72) Inventor: RAYMOND, Phillip Bernard, 5656 AG Eindhoven (NL); YEO, Cheuw Lee, 5656 AG Eindhoven (NL); SANFORD, Braeden, 5656 AG Eindhoven (NL); GUO, Jixiang, 5656 AG Eindhoven (NL); JIANG, Dan, 5656 AG Eindhoven (NL)
(74) Representative: Philips Intellectual Property & Standards
(86) International application number: PCT/EP2023/057454
(87) International publication number: WO 2023/180438

(56) References cited:
- US-A1- 2008 154 503
- US-A1- 2011 310 941
- US-A1- 2013 215 821

## Description

### Field of the Disclosure

The present disclosure is directed generally to private patient monitoring networks, and more specifically, to methods, devices, and systems for establishing a private patient monitoring network over an existing network infrastructure.

### Background

A critical aspect of a large healthcare ecosystem includes maintaining a stable and reliable network infrastructure that connects disparate, discrete, and/or ambulatory instrumentalities within the healthcare ecosystem. For example, in order to take advantage of new technologies, many hospitals have invested significant resources to build a hospital-wide wireless local area network ("WLAN") infrastructure for communication between medical devices, patient monitors, hospital workstations, patient information backend systems, and the like. Nevertheless, healthcare facilities may also be required to invest resources to deploy one or more private network solutions in addition to its existing network infrastructure, which can require a significant up-front capital expenditure to scale-up to the appropriate size for corresponding healthcare facility and/or ecosystem.

US 2011/310941 A1 relates to a radio transceiver system for a mobile communications network. The system comprises a radio transmitter and a radio receiver, and an asynchronous packet-based digital input/output for connecting the remotely located radio transceiver system to at least one digital processing unit.

US 2013/215821 A1 relates to a radio communication system comprising at least a radio access point terminal, a radio relay terminal, and a radio adapter terminal.

US2008/0154503 A1 discloses a method for paging/finding a wireless patient-monitoring device in a WLAN network.

### Summary of the Disclosure

The invention is defined by claims 1 and 5.

The present disclosure is directed generally to devices, systems, and methods involving private patient monitoring networks. More specifically, the present disclosure is directed to devices used to establish a private patient monitoring network over an existing network infrastructure, systems including such private patient monitoring networks, and methods of establishing such network infrastructures.

According to an embodiment of the present disclosure, a pluggable radio adapter for use in a private patient monitoring network system is provided. In aspects, pluggable radio adapter can comprise: at least a first communication radio configured to send and receive data within a first radio frequency ("RF") range; a radio controller operatively connected to at least the first communication radio, wherein the radio controller is configured to process and relay data through the pluggable radio adapter; and a device interface operatively connected to the radio controller, wherein the device interface is configured to operatively connect to the pluggable radio adapter to an associated network access point and to relay data between the pluggable radio adapter and the associated network access point.

In an aspect, the pluggable radio adapter can further comprise at least a second communication radio operatively connected to the radio controller and configured to send and receive data within a second RF range, wherein the second RF range is different from the first RF range.

In an aspect, the pluggable radio adapter can further comprise at least a third communication radio operatively connected to the radio controller and configured to send and receive data within a third RF range, wherein the third RF range is different from the first and second RF ranges.

In an aspect, the first RF range can be from about 1.395 GHz to about 1.4 GHz, from about 1.427 GHz to about 1.432 GHz, and/or from about 1.39 GHz to about 1.395 GHz, from about 1.432 GHz to about 1.435 GHz, the second RF range can be from about 2.402 GHz to about 2.48 GHz, and the third RF range can be from about 2.36 GHz to about 2.4 GHz or from about 2483.5 MHz to about 2500 MHz.

In an aspect, at least the first RF range can be within one of the following ranges: about 608 to about 614 MHz; about 1390 MHz to about 1395 MHz; about 1395 MHz to about 1400 MHz, about 1427 MHz to about 1432 MHz; and/or about 1432 MHz to about 1435 MHz.

In an aspect, the radio controller can be configured to relay data from at least the first communication radio, through the device interface, and to the associated network access point as serial data.

In an aspect, the associated network access point can be a wireless local area network access point.

According to another embodiment of the present disclosure, a private patient monitoring network system is provided. The system can comprise: a network infrastructure comprising a plurality of network access points; and a plurality of pluggable radio adapters, where each pluggable radio adapter (302) is operatively connected to a corresponding network access point. Each pluggable radio adapter can comprise: at least a first communication radio configured to send and receive data within a first radio frequency ("RF") range; a radio controller operatively connected to at least the first communication radio, wherein the radio controller is configured to process and relay data through the pluggable radio adapter; and a device interface operatively connecting the pluggable radio adapter to the corresponding network access point, wherein the device interface is configured to relay data between the pluggable radio adapter and the corresponding network access point.

In an aspect, the private patient monitoring network system can further comprise: a patient monitoring backend system operatively connected to the network infrastructure of the private patient monitoring network system; and one or more end devices in wireless communication with one or more of the plurality of network access points. Each end device can be configured to send and receive data from the one or more network access points within at least the first RF range of the pluggable radio adapter that is connected to each of the one or more network access points.

In an aspect, the radio controller of each pluggable radio adapter can be configured to relay data from at least the first communication radio, through the device interface of the pluggable radio adapter, and to the corresponding network access point as serial data.

In an aspect, each pluggable radio adapter can further comprise: a second communication radio operatively connected to the radio controller and configured to send and receive data within a second RF range, wherein the second RF range is different from the first RF range; and a third communication radio operatively connected to the radio controller and configured to send and receive data within a third RF range, wherein the third RF range is different from the first and second RF ranges.

In an aspect, each network access point of the plurality of network access points can comprise: at least one processor; a network interface; an input/output ("I/O") interface operatively connecting a corresponding pluggable radio adapter to the network access point; and a computer-readable memory storing a radio adapter component. The radio adapter component can include instructions that, when executed by the at least one processor, causes the network access point to perform one or more of the following: establish an end-to-end data path between at least one end device and a backend system, wherein the end-to-end data path includes at least a first wireless communication link between the end device and the network access point, and a second communication link between the network access point and the backend system, the first communication link using a network protocol corresponding to the first RF range and the second communication link using at least one of an IEEE 802.3 network protocol and an IEEE 802.11 network protocol; receiving, via the first communication link of the established end-to-end data path, data from the at least one end device using the pluggable radio adapter; and transmitting the data received from the at least one end device, using the network interface, to the backend device via the second communication link of the established end-to-end data path.

According to yet another embodiment of the present disclosure, a method of establishing a private patient monitoring network system is provided. The method can comprise: (a) providing a network infrastructure comprising a plurality of network access points; (b) connecting a plurality of pluggable radio adapters to the plurality of network access points, wherein each pluggable radio adapter comprises: (i) a first communication radio configured to send and receive data within a first radio frequency ("RF") range; (ii) a radio controller operatively connected to the first communication radio, wherein the radio controller is configured to process and relay data through the pluggable radio adapter; and (iii) a device interface operatively connecting the pluggable radio adapter to a corresponding network access point, the device interface being configured to relay data between the pluggable radio adapter and the corresponding network access point; and (c) establishing at least one end-to-end data path between at least one end device and a backend system, wherein the end-to-end data path includes at least a first communication link between the end device and a corresponding network access point, and a second communication link between the network access point and the backend system, the first communication link using a network protocol corresponding to the first RF range and the second communication link using at least one of an IEEE 802.3 network protocol and an IEEE 802.11 network protocol.

In an aspect, the first RF range can be within one of the following ranges: about 608 MHz to about 614 MHz; about 1390 MHz to about 1395 MHz; about 1395 MHz to about 1400 MHz; about 1427 MHz to about 1432 MHz; and/or about 1432 MHz to about 1435 MHz.

In an aspect, a radio adaptor component can be executed on each network access point of the plurality of network access points, where the radio adapter component operates an adapter management component instance within a network access point host container on the network access point.

These and other aspects of the various embodiments will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the various embodiments.
FIG. 1 is a schematic block diagram illustrating a private network solution for patient monitoring in accordance with some aspects of the present disclosure.
FIG. 2 is another schematic block diagram illustrating a private network solution for patient monitoring in accordance with some aspects of the present disclosure.
FIG. 3 is a schematic block diagram illustrating a private patient monitoring network system according to aspects of the present disclosure.
FIG. 4A is a schematic diagram of pluggable radio adapter illustrated according to aspects of the present disclosure.
FIG. 4B is a schematic diagram of pluggable radio adapter illustrated according to additional aspects of the present disclosure.
FIG. 5 is a block diagram of a network access point including a radio adapter component illustrated according to aspects of the present disclosure.
FIG. 6 is a schematic block diagram of a software interface between a multi-radio pluggable adapter and a network access point host illustrated according to aspects of the present disclosure.
FIG. 7 is a schematic block diagram of a networked access point host software instance for accessing a pluggable radio adapter illustrated according to aspects of the present disclosure.
FIG. 8 is a flowchart illustrating a method of establishing a private patient monitoring network system illustrated according to aspects of the present disclosure.

### Detailed Description of Embodiments

The present disclosure is directed to devices used to establish a private patient monitoring network over an existing network infrastructure, systems including such private patient monitoring networks, and methods of establishing such network infrastructures.

In conventional approaches, healthcare facilities, like hospitals, might deploy a private network solution that requires dedicated access points and access point controllers that interface with the hospital's existing network infrastructure (e.g., the hospital's local area network). Private network solutions can be extremely desirable because they have the ability to deliver highly secure, low latency network and endpoint solutions not currently available with many traditional networks. However, building up such a private network solution typically requires a significant up-front capital expenditure to scale up the private network solution to the appropriate size, despite having already invested resources to build its existing network infrastructure. According to aspects of the present disclosure, the devices, systems, and methods described herein allow for the deployment of a secure and private network solution without its own dedicated network infrastructure by leveraging the healthcare facility's existing network installation.

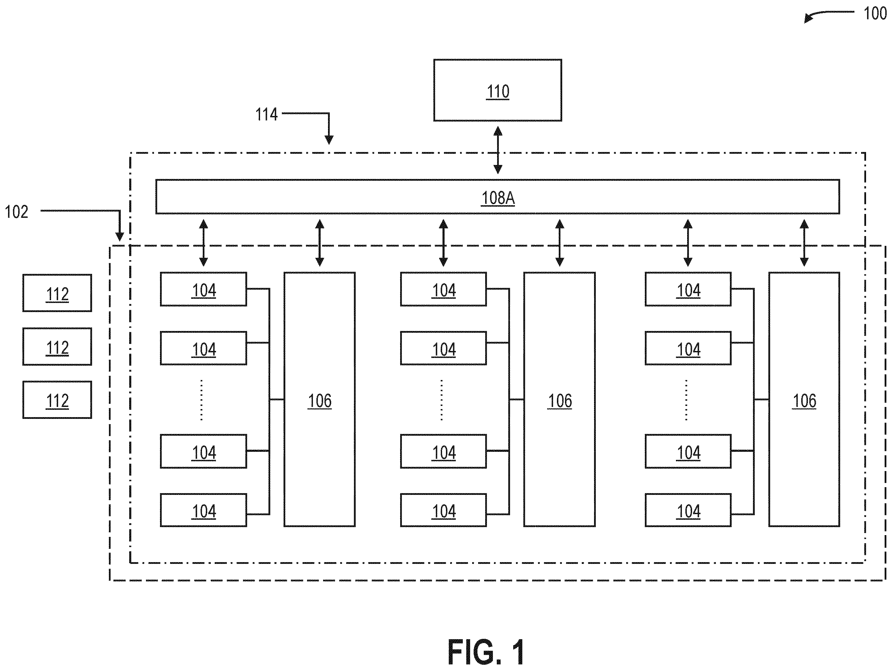

Turning now to FIG. 1, a schematic block diagram of a private network system 100 utilizing a dedicated private network infrastructure 102 is illustrated.

As shown, the dedicated private network 102 comprises multiple dedicated nodes 104, also referred to access points or APs, which can be installed across different buildings, wings, floors, and spaces of a healthcare facility. Each dedicated node / access point 104 within the dedicated private network 102 will be associated with a dedicated node controller 106, also referred to as an access point controller as APC. Each access point controller 106 can manage multiple access points 104. As shown, the APs 104 and APCs 106 can be connected to the facility's existing network infrastructure 108A.

Typically, the existing network infrastructure 108A of a hospital may be a local area network ("LAN") that facilitates data communication via a network protocol such as the IEEE 802.3 and IEEE 802.11 network protocols. In embodiments, the existing network infrastructure 108A can also be connected to one or more backend systems, such as a backend system 110 used for patient monitoring and data collection. In embodiments, the backend system 110 can be a centralized patient information center, such as the Philips IntelliVue^{®} Information Center (also referred to as PIIC iX).

One or more end user devices 112 can be configured to connect to one access point 104 at a time via a dedicated private communication link. That is, each access point 104 within the dedicated private network 102 can include a specialized radio frequency ("RF") transceiver for connecting with one or more end user devices 112 that utilize the same dedicated RF communications technology.

In specific examples, the end user devices 112 can be one or more types of patient monitors, including but not limited to a Philips IntelliVue^{®} patient monitor. For example, the end user devices 112 may be a wearable and/or wireless patient monitor, such as the Philips IntelliVue^{®} MX40.

In embodiments, communication between the end devices 112 and the dedicated private network 102 (i.e., the access points 104) may operate on the protected ~1.4 GHz Wireless Medical Telemetry Service ("WMTS") and adjacent spectrum (e.g., the TerreStar E-WMTS spectrum, which includes from about 1.39 GHz to about 1.395 GHz, and from about 1.432 GHz to about 1.435 GHz). In other embodiments, communication between the end devices 112 and the dedicated private network 102 (i.e., the access points 104) may operate within the ~2.4 GHz and/or the Medical Body Area Network ("MBAN") spectrum (e.g., from about 2.36 GHz to about 2.4 GHz, or from about 2.4835 GHz to about 2.5 GHz).

Together, the dedicated private network 102 and the existing network infrastructure 108A constitute a patient monitoring network system that enables an end-to-end data path between a backend system 110 and an end user device 112 with the access point 104 and access point controller 106 using an IP-based routing protocol to forward data from the end device(s) 112 to the backend system 110.

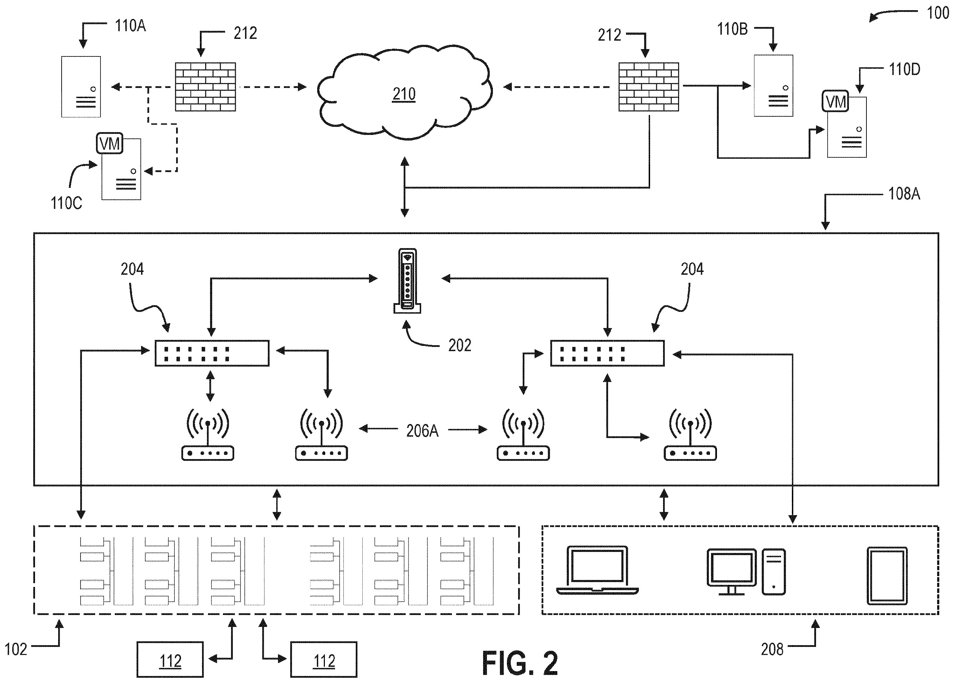

With reference to FIG. 2, a schematic block diagram of a private network system 100 utilizing a dedicated private network infrastructure 102 in addition to an existing network infrastructure 108A is illustrated.

In certain aspects, the existing network infrastructure 108A can include a wired or Ethernet LAN, a wireless LAN ("WLAN"), or a combination thereof. The existing network infrastructure 108A can be variously embodied as known in the art, but generally includes a combination of modems 202, switches 204, and WLAN access points 206A operatively connected to one another. It will be appreciated that the private network system 100 and the existing network infrastructure 108A can include, in various forms (i.e., with one or more physical and/or digital components), one or more security features, such as firewalls 212. In embodiments, the WLAN access points 206A can be configured to include such security features.

The network access points 206A may be primarily configured to connect with one or more user devices 208, such as computers, laptops, workstations, tablets, mobile phones, and the like. By connecting with a WLAN access point 206A, the user devices 208 can be connected to a communications network 210, such as the Internet.

In embodiments, the existing network infrastructure 108A can facilitate communication between one or more user devices 208 and one or more backend systems 110, such as a remote backend system 110A accessible through the communications network 210 or a local backend system 110B accessible directly through the existing network 108A. As used herein, the term "remote... system" refers to an electronic system that is located off-site from the existing network 108A, regardless of whether the device is physical or virtual in nature (e.g., a physical system 110A or a virtual machine 110C). In contrast, the term "local... system" refers to an electronic system that is located on-site with the existing network 108A, regardless of whether the device is physical or virtual in nature (e.g., a physical system 110B or a virtual machine 110D).

Thus, in addition to an existing network infrastructure 108A, the private network system 100 also includes a dedicated private network infrastructure 102 built up separately from the existing network infrastructure 108A. However, building up such a dedicated private network 102 to a scale that is useful within most healthcare ecosystems requires significant planning and costs.

Accordingly, described herein are pluggable radio adapters 302 that use the existing network infrastructure 108B (e.g., a hospital-wide WLAN network infrastructure) as a hosting network infrastructure to deploy a private patient monitoring network system 300.

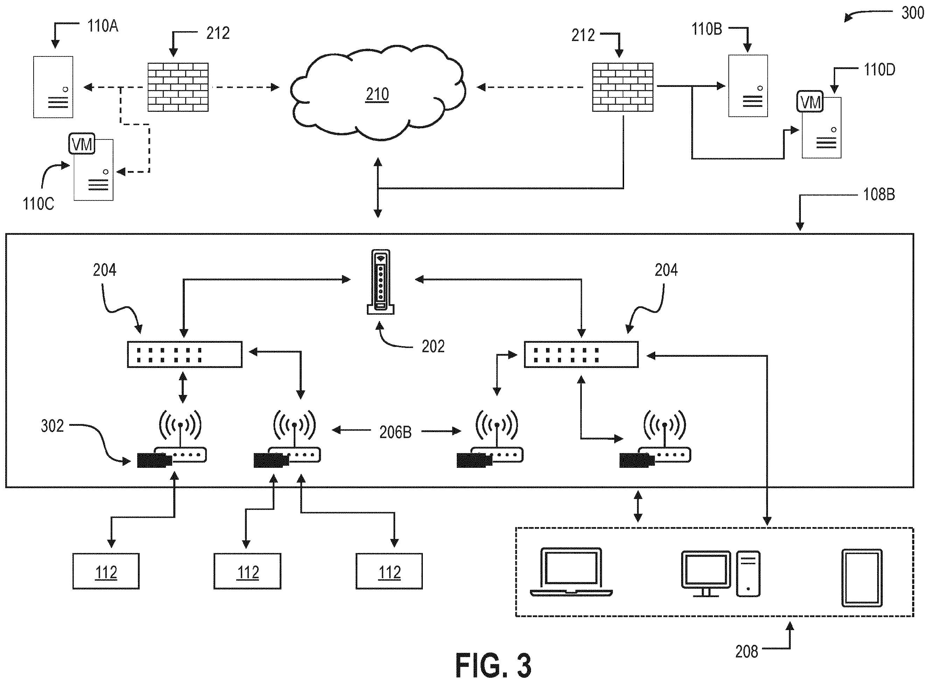

With reference to FIG. 3, a private patient monitoring network system 300 is illustrated according to certain aspects of the present disclosure. As shown, the private patient monitoring network system 300 can include a local area network 108B, which can be an existing network infrastructure as described above (e.g., existing network 108A), wherein the local area network 108B comprises a plurality of wireless local area network ("WLAN") access points 206B configured to operatively receive a pluggable radio adapter 302. By using the existing WLAN infrastructure 108A, 108B, an end-to-end data path can be established to support communications between end devices 112 and a backend system 110 (e.g., backend systems 110A-110D).

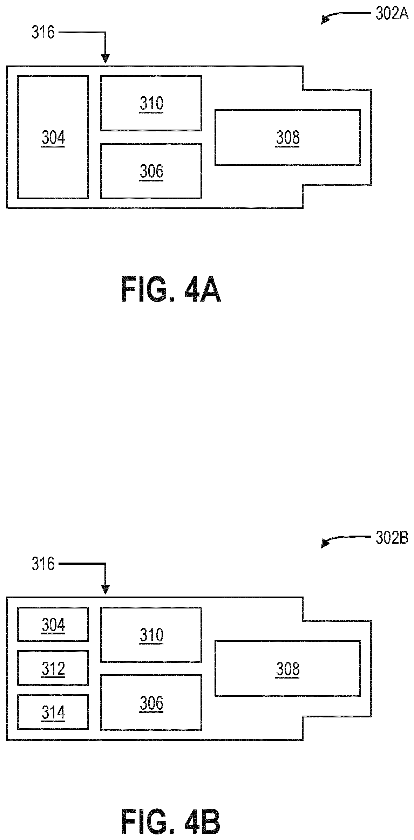

With further reference to FIGS. 4A and 4B, such pluggable radio adapters 302 are illustrated according to certain embodiments of the present disclosure. In one embodiment, the pluggable radio adapter 302A, 302B can include at least a first communication radio 304, a radio controller 306 operatively connected to at least the first communication radio 304, and a device interface 308 operatively connecting the pluggable radio adapter 302A, 302B to a corresponding WLAN access point 206B. In further embodiments, the pluggable radio adapter 302A, 302B can include a peripheral interface 310. In still further embodiments, the pluggable radio adapter 302B can include at least a second communication radio 312 configured to operate within a second RF range, including at least a third communication radio 314 configured to operate within a third RF range. Each of these components are discussed in more detail below.

According to certain embodiments of the present disclosure, the pluggable radio adapter 302 can include a single communication radio 304 (e.g., pluggable radio adapter 302A). In other embodiments, the pluggable radio adapter 302 can include two or more communication radios 304, 312, 314 communication radio(e.g., pluggable radio adapter 302B). Each of the one or more communication radios 304, 312, 314 of the pluggable radio adapter 302A, 302B can be configured to send and receive data within a predefined portion of the RF spectrum (i.e., within a particular RF range). For example, each of the communication radios 304, 312, 314 can facilitate wireless communication between an end device 112 and the pluggable radio adapter 302A, 302B via a wireless communication link that is based on a network protocol corresponding to the particular RF range employed. In order to achieve this, each communication radio 304, 312, 314 contains hardware and circuitry, such as antennas, configured to radiate and/or receive radio waves at the designated frequencies.

In some aspects, at least a first communication radio 304 can be configured to send and receive data within a first radio frequency ("RF") range. In some embodiments, the first RF range can include one or more of the following ranges: from about 608 to about 614 MHz; from about 1390 MHz to about 1395 MHz; from about 1395 MHz to about 1400 MHz; from about 1427 MHz to about 1432 MHz; and/or from about 1432 MHz to about 1435 MHz.

In further aspects, at least a second communication radio 312 can be configured to send and receive data within a second RF range that is different from the first RF range of the first communication radio 304. In some embodiments, the second RF range can be from about 2.402 GHz to about 2.48 GHz. In other embodiments, the second RF range can be from about 608 MHz to about 614 MHz, or from about 1395 MHz to about 1400 MHz, or from about 1427 MHz to about 1432 MHz. In specific embodiments, the second communication radio 312 can be a Bluetooth radio.

In still further aspects, at least a third communication radio 314 can be configured to send and receive data within a third RF range that is different from the first RF range and the second RF range. In some embodiments, the third RF range can be from about 2.36 GHz to about 2.4 GHz, or from about 2483.5 MHz to about 2500 MHz depending on geography and other considerations. In other embodiments, the third RF range can be from about 608 MHz to about 614 MHz, from about 1395 MHz to about 1400 MHz, from about 1427 MHz to about 1432 MHz. In embodiments, one or more of the RF ranges can include the TerreStar E-WMTS spectrum (e.g., from about 1390 MHz to about 1395 MHz, from about 1432 MHz to about 1435 MHz). In specific embodiments, the third communication radio 314 can be a Medical Body Area Network (MBAN) radio.

According to certain embodiments of the present disclosure, the pluggable radio adapter 302 can include a radio controller 306 operatively connected to the communication radios 304, 312, 314. In an aspect, the radio controller 306 may be configured to process and relay data through the pluggable radio adapter 302A, 302B.

More specifically, the radio controller 306 can be used to run a protocol stack firmware that, when plugged into a corresponding WLAN access point 206B, exposes the pluggable radio adapter 302A, 302B to an adapter management component (530) of the corresponding WLAN access point 206B (discussed further below).

In some embodiments, the radio controller 306 can be a field programmable gate array ("FPGA") specifically programmed to facilitate communications through the pluggable radio adapter 302A, 302B using one or more specific communication radios 304, 312, 314. The FPGA can include programmable logic components called "logic blocks" as well as programmable interconnects, which can be programmed after manufacturing by a customer or designer to implement one or more features needed to operate the pluggable radio adapter 302. An FPGA's logic blocks can be programmed to perform the operations of basic logic gates, or more complex combinational operators, and typically include memory elements, which may be individual circuit flip-flops or more complex blocks of memory. In other embodiments, the radio controller 306 can be a microcontroller containing one or more processors, a memory storing instructions for operating the pluggable radio adapter 302A, 302B, and an input/output interface able to connect with peripheral devices (e.g., the communication radios 304, 312, 314).

According to certain embodiments of the present disclosure, each pluggable radio adapter 302 can include a device interface 308 operatively connected to the radio controller 306 and configured to operatively connect the pluggable radio adapter 302 to a corresponding network access point 206B.

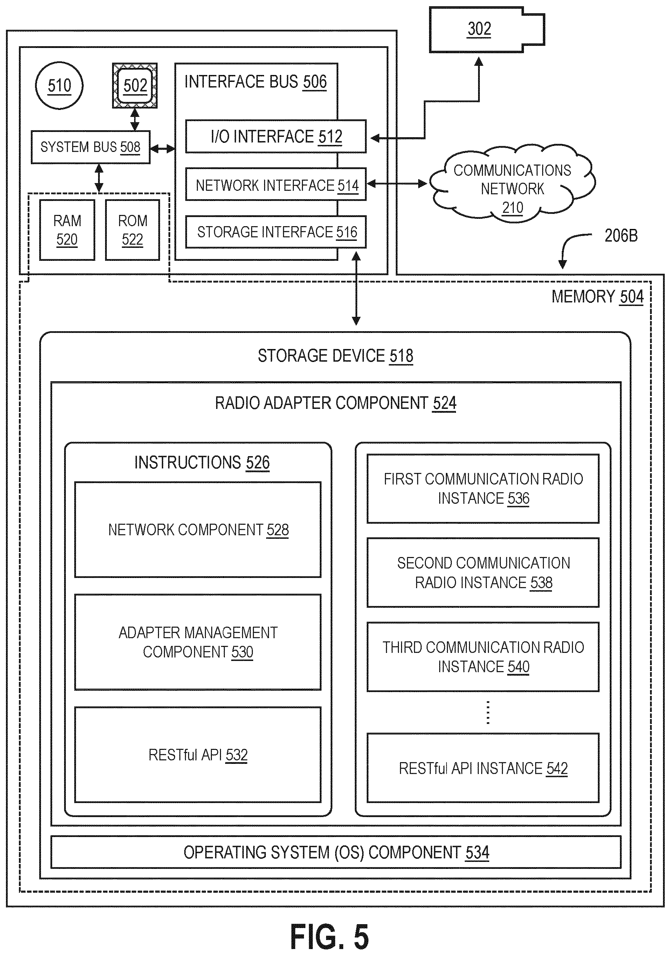

In particular embodiments, the device interface 308 can expose the pluggable radio adapter 302 to the corresponding network access point 206B as a universal asynchronous receiver-transmitter ("UART") or serial device, so that the adapter management component 530 (shown in FIG. 5) of the corresponding network access point 206B can control the communication radios 304, 312, 314 using a standard serial protocol. In aspects, if a pluggable radio adapter 302 contains multiple communication radios 304, 312, 314 (e.g., adapter 302B), each radio 304, 312, 314 within the adapter 302B can be attached to the network access point 206B as a separate serial device via the device interface 308.

According to some embodiments of the present disclosure, each pluggable radio adapter 302 can also include a peripheral interface 310 that connects the pluggable radio adapter 302 to an interface of the network access point 206B. The peripheral interface 310 contains conductive electrical circuits / pathways that facilitate the transmission of data from the pluggable radio adapter 302 to the network access point 206B. In an aspect, the peripheral interface 310 can be a USB interface that connects the pluggable radio adapter 302 to a host USB interface of the network access point 206B. In specific aspects, peripheral interface 310 can include a USB 2.0 interface, a USB 3.0 interface, a USB Type A interface, a USB Type B interface, a micro-USB interface, a USB Type C interface, a thunderbolt interface, and/or the like.

According to various aspects of the present disclosure, the pluggable radio adapter 302 can be provided within a device housing 316 that has the form-factor of a USB device. In other words, the device housing 316 can enclose the components of the pluggable radio adapters 302 described herein and have dimensions less than about 10 cm in length, less than about 5 cm in width, and less than about 5 cm in height, including less than about 5 cm in length, less than about 3 cm in width, and less than about 3 cm in height.

Turning now to FIG. 5, additional features of the network access points 206B of a private patient monitoring network system 300 are illustrated according to certain aspects of the present disclosure. In embodiments, the network access point 206B can comprise at least one processor 502 (also referred to as central processing units or CPUs), machine-readable memory 504, an interface bus 506, all of which may be interconnected and/or communicate through a system bus 508 containing conductive circuit pathways through which instructions (e.g., machine-readable signals) may travel to effectuate communications, tasks, storage, and the like. The network access point 206B may be connected to a power source 510, which can include an internal power source and/or an external power source.

In embodiments, the at least one processor 502 can comprise a high-speed data processor adequate to execute program components, which may include various specialized processing units as may be known in the art. The general processor may be a microprocessor, or may also be any traditional processor, controller, microcontroller, or state machine. In some embodiments, one or more of the features described herein may be implemented on components such as an Application-Specific Integrated Circuit ("ASIC"), a Digital Signal Processor ("DSP"), a Field Programmable Gate Array ("FPGA"), or similar electronics.

In embodiments, the interface bus 506 may include an input/output interface 512 configured to connect the network access point 206B to one or more peripheral devices (e.g., a pluggable radio adapter 302 via a serial data protocol), a network interface 514 configured to connect the network access point 206B to a communications network 210 (e.g., using a network protocols such as IEEE 802.3 and/or 802.11), and/or a storage interface 516 configured to accept, communicate, and/or connect to a number of machine-readable memory devices (e.g., storage device 518, removable storage devices, etc.).

In aspects, the input/output ("I/O") interface 512 can accept, communicate, or otherwise connect an input device, and may employ connection protocols such as, but not limited to, IEEE 1394a-b, serial, universal serial bus ("USB"), and the like. In particular embodiments, the I/O interface 512 of a network access point 206B operatively connects the network access point 206B to a corresponding pluggable radio adapter 302.

In aspects, the network interface 514 operatively connects the network access point 206B to a communications network 210, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some embodiments, one or more user devices (e.g., user devices 208) may connect with the network access point 206B via the communications network 210 and the network interface 514.

In embodiments, the memory 504 can be variously embodied in one or more forms of machine-accessible and machine-readable memory, including a various types of storage devices 518, random access memory 520, and read-only memory 522. In aspects, the storage device 518 can include a non-transitory storage medium, a magnetic disk storage, an optical disk storage, an array of storage devices, a solid-state memory device, and the like, including combinations thereof.

As shown in FIG. 5, the memory 504 can include a radio adapter component 524 that includes a collection of program and/or database components and/or data. Depending on the particular implementation, the radio adapter component 524 may include software components, hardware components, and/or some combination of both hardware and software components.

In particular embodiments, the radio adapter component 524 can include, but is not limited to, instructions 526 having a network component 528, an adapter management component 530, a RESTful application programming interface ("API") component 542, and/or an operating system component 534.

The aforementioned components may be incorporated into, loaded from, loaded onto, or otherwise operatively available to and from the radio adapter component 524. Similarly, the radio adapter component 524 can be incorporated into, loaded from, loaded onto, or otherwise operatively available to and from the network access point 206B. For example, although program components may be stored in a local storage device 518, they may also be loaded and/or stored in other memory, such as a remote cloud storage facility accessible through a communications network (e.g., communications network 210).

The operating system component 534 can be an executable program component facilitating the operation of the network access point 206B. Typically, the operation system component 534 may facilitate access of the I/O, network, and storage interfaces, and may communicate with other components of the system.

The network component 528 can be a stored program component that is executed by at least one processor, such as the at least one processor 502 of the network access point 206B. In particular, the network component 528 can facilitate communications to and from the network access point 206B via the network interface 514 in accordance with standard network protocols such as IEEE 802.3, 802.11, and the like.

More specifically, the network component 528 can negotiate communications through the existing network infrastructure 108B in order to connect one or more peripheral devices to, for example, a backend system 110 of a private patient monitoring network system 300. In other words, the network component 528 is responsible for establishing a communication link between the network access point 206B and one or more backend systems 110. As described above, the communication link can include a wired communication link, a wireless communication link, or a combination thereof.

The adapter management component 530 can be a stored program component that is executed by at least one processor, such as the at least one processor 502 of the network access point 206B. In particular embodiments, the adapter management component 530 is configured to run on the network access point 206B and manage the corresponding pluggable radio adapter 302 in both the control and data planes. In some embodiments, the pluggable radio adapter 302 may be exposed to the network access point 206B through the adapter management component 530 as a UART or serial device such that the adapter management component 530 can control the communication radio(s) 304, 312, 314 of the corresponding pluggable radio adapter 302 using a standard serial data protocol.

In embodiments, the adapter management component 530 may operate the network access point 206B using multiple instances 536, 538, 540 to separately manage each radio interface of the pluggable radio adapter 302 in both the control and data planes. For example, as discussed above, the pluggable radio adapter 302 can be exposed to the network access point 206B through separate UART or serial interfaces, so that each communication radio 304, 312, 314 can be attached as a separate serial device in the network access point 206B.

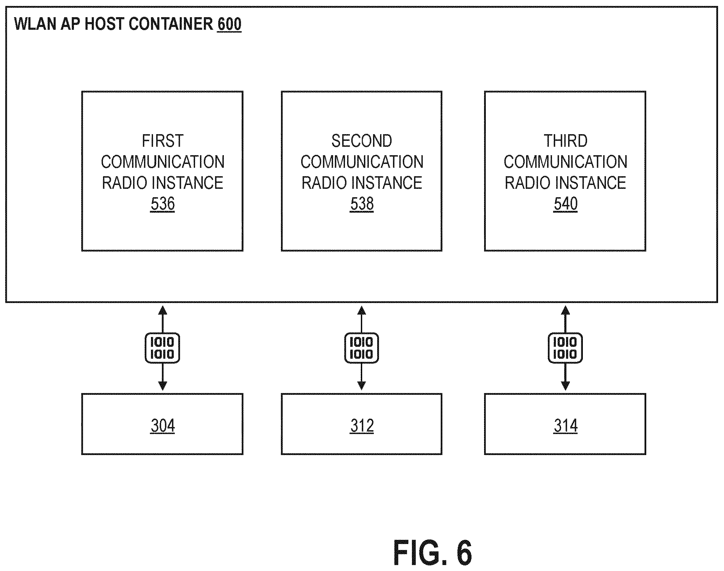

In certain aspects, the radio adapter component 524 of the network access point 206B may support a container-based software environment. For example, as shown in FIG. 6, inside a network access point 206B host container 600, the adapter management component 530 can run inside separate container instances 536, 538, 540 for each radio 304, 312, 314 respectively, with each radio 304, 312, 314 attached to the host container 600 as a separate serial device.

In further aspects, the adapter management component 530 can include Linux BlueZ based software stack for the Bluetooth LE radio, which manages the Bluetooth LE radio (e.g., communication radio 312 in some embodiments) in both the control and data planes. In some aspects, the adapter management component 530 can also include an MBAN software stack for an MBAN radio (e.g., IEEE 802.15.4 radio operating in the MBAN frequency with OpenThread stack in the upper layer for an end-to-end IPv6 mesh network).

In embodiments, the network access point 206B does not have a container hosting capability. In such embodiments, the network access point 206B can include a RESTful API component 542 configured to send and receive data received via the pluggable radio adapter 302. Thus, in such embodiments, one or more parts of the adapter management component 530 may be run from a virtual machine server in the existing network 108B.

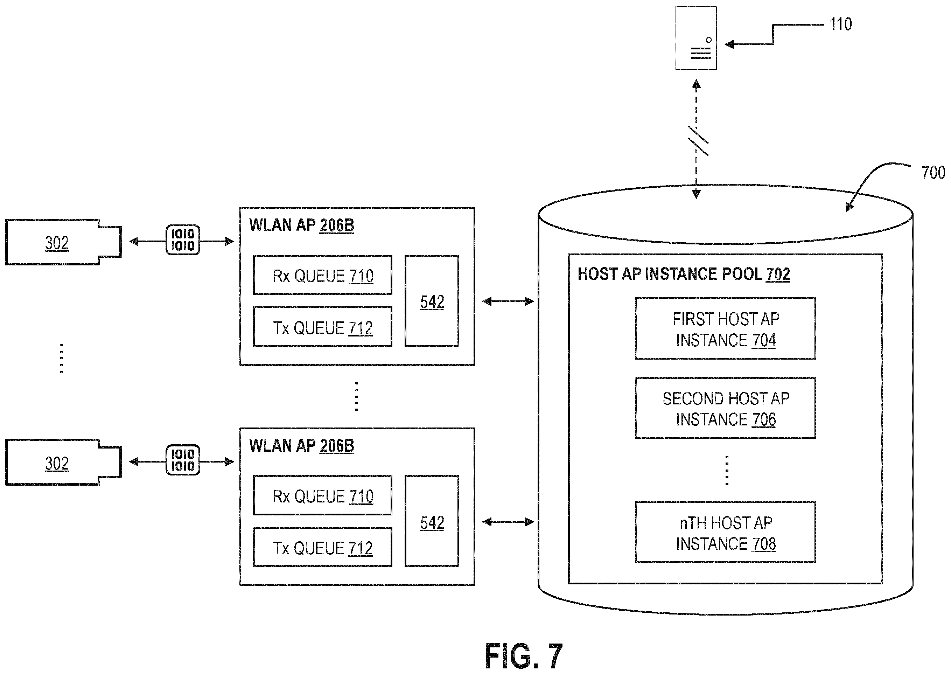

For example, as shown in FIG. 7, the adapter management component 530 is not operated in the network access point 206B but in a virtual machine server 700 in the existing network 108B, where it creates a pool 702 of host access point instances 704, 706, 708 (i.e., an instance of the adapter management component 530) for each pluggable radio adapter 302. Each pluggable radio adapter 302 can be assigned a host access point instance 704, 706, 708, which can use the RESTful network API to read and/or write data from or into the corresponding pluggable radio adapter 302. On each network access point 206B, a corresponding pluggable radio adapter 302 can be connected as a serial device, where the RESTful API 542 is run, which maintains a receiver queue 710 and a transmitter queue 712 for data being received and transmitted, respectively.

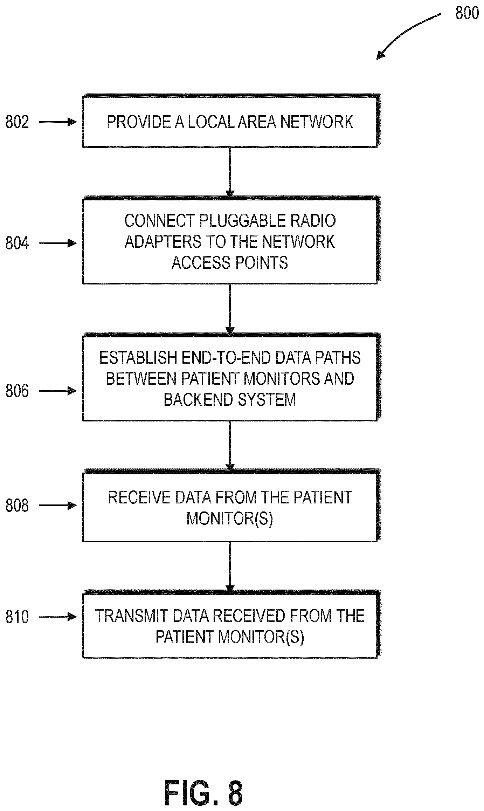

Also disclosed herein are methods 800 of establishing a private patient monitoring network system 300. With reference to FIG. 8, a method 800 of establishing a private patient monitoring network system 300 is illustrated according to certain aspects of the present disclosure.

At a step 802, the method 800 can include providing or acquiring an existing network infrastructure 108A, 108B that includes a plurality of network access points 206A, 206B. In embodiments, the network infrastructure 108A, 1008B provided can be a local area network and the plurality of network access points 206A, 206B can include wireless local area network access points.

At a step 804, the method 800 can include connecting a plurality of pluggable radio adapters 302 to the plurality of network access points 206A, 206B. In other words, an individual pluggable radio adapter 302 can be connected to a single corresponding network access point 206A, 206B of the plurality of network access point 206A, 206B.

As described above, each of the pluggable radio adapters 302 can include: at least a first communication radio 304 configured to send and receive data within a first radio frequency ("RF") range; a radio controller 306 operatively connected to the first communication radio 304, wherein the radio controller 306 is configured to process and relay data through the pluggable radio adapter 302; and a device interface 308 operatively connecting the pluggable radio adapter 302 to a corresponding network access point 206B, the device interface 308 being configured to relay data between the pluggable radio adapter 302 and the corresponding network access point 206B.

At a step 806, the method 800 can include establishing at least one end-to-end data path between at least one patient monitor 112 and a patient monitoring backend system 110. In embodiments, the end-to-end data path can include at least a first communication link between the patient monitor 112 and a corresponding pluggable radio adapter 302 that is connected to a network access point 206B.

In some embodiments, the first communication link can be a wireless communication link created with the first communication radio 304 of the pluggable radio adapter 302A, 302B using a network protocol corresponding to the first RF range of the first communication radio 304. In other embodiments, the first communication link can also be a wireless communication link created with the second communication radio 312 of the pluggable radio adapter 302B using a network protocol corresponding to the second RF range of the second communication radio 312. In still further embodiments, the first communication link can be a wireless communication link created with the third communication radio 314 of the pluggable radio adapter 302B using a network protocol corresponding to the third RF range of the third communication radio 314.

Although three communication radios 304, 312, 314 are explicitly described herein, the pluggable radio adapters 302 can include additional communication radios as desired that correspond to different bands of the RF spectrum.

The end-to-end data path can further include at least a second communication link between the network access point 206B connected to the corresponding pluggable radio adapter 302 and the patient monitoring backend system 110. In particular embodiments, the second communication link includes a wired communication link, a wireless communication, or a combination thereof. In some embodiments, at least a portion of the second communication link is a wireless connection and is created with the network interface 514 of the network access point 206B using an IEEE 802.11 network protocol. In embodiments, at least a portion of the second communication link is a wired connection and is created with the network interface 514 of the network access point 206B using an IEEE 802.3 network protocol. However, other components and devices, including various virtual machines and firewalls, whether wired or wireless, may be employed as described herein and/or known in the art.

In embodiments, the method 800 can include a step 808 wherein data is received from at least one end device 112 (e.g., a patient monitor) by the network access point 206B using the connected pluggable radio adapter 302. In other words, step 808 includes receiving data, at the network access point 206B, from at least one end device 112 (e.g., a patient monitor, etc.) using a network protocol corresponding to an RF range specific to one or more communication radios 304, 312, 314 present in the pluggable radio adapter 302. In embodiments, the data is sent from the end device 112 to the network access point 206B over the first communication link of the end-to-end data path established using the pluggable network adapter 302.

In embodiments, the method 800 can also include a step 810 wherein data that is received from the at least one end device 112 in step 808 is then transmitted to at least one backend system 110. In some embodiments, the data is transmitted using the network interface 514 as discussed above. More particularly, the data is sent from the network access point 206B to the backend system(s) 110 via the second communication link of the end-to-end data path established in step 806.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A private patient monitoring network system (300), the private patient monitoring network system (300) comprising:
a network infrastructure (108A, 108B) comprising a plurality of network access points (206A, 206B);
a plurality of pluggable radio adapters (302), each pluggable radio adapter (302) operatively connected to a corresponding network access point (206A, 206B), wherein each pluggable radio adapter (302) comprises:
at least a first communication radio (304, 312, 314) configured to send and receive data within a first radio frequency, RF, range;
a radio controller (306) operatively connected to at least the first communication radio (304, 312, 314), wherein the radio controller (306) is configured to process and relay data through the pluggable radio adapter (302);
a device interface (308) operatively connecting the pluggable radio adapter (302) to the corresponding network access point (206A, 206B), the device interface (308) being configured to relay data between the pluggable radio adapter (302) and the corresponding network access point (206A, 206B);
a patient monitoring backend system (110) operatively connected to the network infrastructure (108A, 108B) of the private patient monitoring network system (300); and
one or more patient monitoring end devices (112) in wireless communication with one or more of the plurality of network access points (206A, 206B), each patient monitoring end device (112) being configured to send and receive data from the one or more of the plurality of network access points (206A, 206B), using the pluggable radio adapters connected to the one or more network access points, within at least the first RF range of the pluggable radio adapter (302) that is connected to each of the one or more of the plurality of network access points (206A, 206B); wherein the private patient monitoring network system is configured to establish at least one end-to-end data path between the at least one patient monitoring end device of the one or more patient monitoring end devices and the patient monitoring backend system, wherein the at least one end-to-end data path includes at least a first communication link between the one of the plurality of network access points and the patient monitoring end device and corresponding pluggable radio adaptor that is connected to one of the plurality of the network access points (206A, 206B), and a second communication link between the one of the plurality of network access points and the patient monitoring backend systems, the first communication link using a network protocol corresponding to the first RF range and the second communication link using either one of: a wired communication link, a wireless communication link, or a combination thereof.

2. The private patient monitoring system (300) of claim 1, wherein the radio controller (306) of each pluggable radio adapter (302) is configured to relay data from at least the first communication radio (304, 312, 314), through the device interface (308) of the pluggable radio adapter (302), and to the corresponding network access point (206A, 206B) as serial data.

3. The private patient monitoring system (300) of any of claims 1-2, wherein each pluggable radio adapter (302, 302B) further comprises:
a second communication radio (304, 312, 314) operatively connected to the radio controller (306) and configured to send and receive data within a second RF range, wherein the second RF range is different from the first RF range; and
a third communication radio (304, 312, 314) operatively connected to the radio controller (306) and configured to send and receive data within a third RF range, wherein the third RF range is different from the first and second RF ranges.

4. The private patient monitoring system (300) of any of claims 1-3, wherein each network access point (206A, 206B) of the plurality of network access points (206A, 206B) comprises:
at least one processor (502);
a network interface (514);
an input/output, I/O, interface (512) operatively connecting a corresponding pluggable radio adapter (302) to the network access point (206A, 206B); and
a computer-readable memory (504) storing a radio adapter component (524), the radio adapter component (524) including instructions that, when executed by the at least one processor, causes the network access point (206A, 206B) to perform one or more of the following:
establish an end-to-end data path between at least one patient monitoring end device (112) and the patient monitoring backend system (110), wherein the end-to-end data path includes at least a first wireless communication link between the end device (112) and a corresponding pluggable radio adaptor that is connected to one of the network access points (206A, 206B), and a second communication link between the network access point (206A, 206B) and the backend system (110), the first communication link using a network protocol corresponding to the first RF range and the second communication link using at least one of an IEEE 802.3 network protocol and an IEEE 802.11 network protocol;
receiving, via the first communication link of the established end-to-end data path, data from the at least one end device (112) using the pluggable radio adapter (302); and
transmitting the data received from the at least one end device (112), using the network interface (514), to the backend device (110) via the second communication link of the established end-to-end data path.

5. A method (800) of establishing a private patient monitoring network system (300), the method (800) comprising:
providing (802) a network infrastructure (108A, 108B) comprising a plurality of network access points (206A, 206B);
connecting (804) a plurality of pluggable radio adapters (302) to the plurality of network access points (206A, 206B), wherein each pluggable radio adapter (302) comprises:
a first communication radio (304) configured to send and receive data within a first radio frequency, RF, range;
a radio controller (306) operatively connected to the first communication radio (304), wherein the radio controller (306) is configured to process and relay data through the pluggable radio adapter (302); and
a device interface (308) operatively connecting the pluggable radio adapter (302) to a corresponding network access point (206A, 206B), the device interface (308) being configured to relay data between the pluggable radio adapter (302) and the corresponding network access point (206A, 206B); and
establishing (806) at least one end-to-end data path between at least one patient monitoring end device (112) and a patient monitoring backend system (110), wherein the at least one end-to-end data path includes at least a first communication link between the at least one patient monitoring end device (112) and a corresponding pluggable radio adaptor that is connected to one of the plurality of the network access points (206A, 206B), and a second communication link between one of the plurality of the network access points (206A, 206B) and the patient monitoring backend system (110), the first communication link using a network protocol corresponding to the first RF range and the second communication link using either one of: a wired communication link, a wireless communication link, or a combination thereof.

6. The method (800) of claim 5, wherein the second communication link using at least one of an IEEE 802.3 network protocol and an IEEE 802.11 network protocol.

7. The method (800) of claim 6, wherein the first RF range is within one of the following ranges: about 608 MHz to about 614 MHz; about 1390 MHz to about 1395 MHz; about 1395 MHz to about 1400 MHz; about 1427 MHz to about 1432 MHz; and/or about 1432 MHz to about 1435 MHz.

8. The method of any of claims 6 or 7, wherein a radio adaptor component (524) is executed on each network access point (206A, 206B) of the plurality of network access points (206A, 206B) that operates an adapter management component (530) instance within a network access point host container (600) on the network access point (206A. 206B).

## Patentansprüche

1. Privates Patientenüberwachungs-Netzwerksystem (300), wobei das private Patientenüberwachungs-Netzwerksystem (300) umfasst:
eine Netzwerkinfrastruktur (108A, 108B), die eine Vielzahl von Netzwerkzugangspunkten (206A, 206B) umfasst;
eine Vielzahl steckbarer Funkadapter (302), wobei jeder steckbare Funkadapter (302) betriebsmäßig mit einem entsprechenden Netzwerkzugangspunkt (206A, 206B) verbunden ist, wobei jeder steckbare Funkadapter (302) umfasst:
zumindest ein erstes Kommunikationsfunkmodul (304, 312, 314), das so konfiguriert ist, dass es Daten innerhalb eines ersten Funkfrequenz- (radio frequency, RF-) Bereichs sendet und empfängt;
eine Funksteuerung (306), die betriebsmäßig mit zumindest dem ersten Kommunikationsfunkmodul (304, 312, 314) verbunden ist, wobei die Funksteuerung (306) so konfiguriert ist, dass sie Daten verarbeitet und über den steckbaren Funkadapter (302) weiterleitet;
eine Geräteschnittstelle (308), die den steckbaren Funkadapter (302) betriebsmäßig mit dem entsprechenden Netzwerkzugangspunkt (206A, 206B) verbindet, wobei die Geräteschnittstelle (308) so konfiguriert ist, dass sie Daten zwischen dem steckbaren Funkadapter (302) und dem entsprechenden Netzwerkzugangspunkt (206A, 206B) weiterleitet;
ein Patientenüberwachungs-Backend-System (110), das betriebsmäßig mit der Netzwerkinfrastruktur (108A, 108B) des privaten Patientenüberwachungs-Netzwerksystems (300) verbunden ist; und
ein oder mehrere Patientenüberwachungs-Endgeräte (112), die in drahtloser Kommunikation mit einem oder mehreren der Vielzahl von Netzwerkzugangspunkten (206A, 206B) stehen, wobei jedes Patientenüberwachungs-Endgerät (112) so konfiguriert ist, dass es unter Verwendung der steckbaren Funkadapter, die mit dem einen oder den mehreren Netzwerkzugangspunkten verbunden sind, Daten innerhalb zumindest des ersten RF-Bereichs des jeweils mit dem einen oder den mehreren der Vielzahl von Netzwerkzugangspunkten (206A, 206B) verbundenen steckbaren Funkadapters (302) an den einen oder die mehreren der Vielzahl von Netzwerkzugangspunkten (206A, 206B) sendet und von diesen empfängt; wobei das private Patientenüberwachungsnetzwerksystem so konfiguriert ist, dass es mindestens einen Ende-zu-Ende-Datenpfad zwischen dem mindestens einen Patientenüberwachungs-Endgerät des einen oder der mehreren Patientenüberwachungs-Endgeräte und dem Patientenüberwachungs-Backend-System einrichtet, wobei der mindestens eine Ende-zu-Ende-Datenpfad zumindest eine erste Kommunikationsverbindung zwischen dem einen der Vielzahl von Netzwerkzugangspunkten und dem Patientenüberwachungs-Endgerät und dem entsprechenden steckbaren Funkadapter umfasst, der mit einem der Vielzahl von Netzwerkzugangspunkten (206A, 206B) verbunden ist, sowie eine zweite Kommunikationsverbindung zwischen dem einen der Vielzahl von Netzwerkzugangspunkten und den Patientenüberwachungs-Backend-Systemen umfasst, wobei die erste Kommunikationsverbindung ein Netzwerkprotokoll verwendet, das dem ersten RF-Bereich entspricht, und die zweite Kommunikationsverbindung entweder eine drahtgebundene Kommunikationsverbindung, eine drahtlose Kommunikationsverbindung oder eine Kombination aus diesen verwendet.

2. Privates Patientenüberwachungssystem (300) nach Anspruch 1, wobei die Funksteuerung (306) jedes steckbaren Funkadapters (302) so konfiguriert ist, dass sie Daten von zumindest dem ersten Kommunikationsfunkmodul (304, 312, 314) über die Geräteschnittstelle (308) des steckbaren Funkadapters (302) an den entsprechenden Netzwerkzugangspunkt (206A, 206B) als serielle Daten weiterleitet.

3. Privates Patientenüberwachungssystem (300) nach einem der Ansprüche 1-2, wobei jeder steckbare Funkadapter (302, 302B) ferner umfasst:
ein zweites Kommunikationsfunkmodul (304, 312, 314), das betriebsmäßig mit der Funksteuerung (306) verbunden und so konfiguriert ist, dass es Daten innerhalb eines zweiten RF-Bereichs sendet und empfängt, wobei sich der zweite RF-Bereich vom ersten RF-Bereich unterscheidet; und
ein drittes Kommunikationsfunkmodul (304, 312, 314), das betriebsmäßig mit der Funksteuerung (306) verbunden und so konfiguriert ist, dass es Daten innerhalb eines dritten RF-Bereichs sendet und empfängt, wobei sich der dritte RF-Bereich vom ersten und zweiten RF-Bereich unterscheidet.

4. Privates Patientenüberwachungssystem (300) nach einem der Ansprüche 1-3, wobei jeder Netzwerkzugangspunkt (206A, 206B) der Vielzahl von Netzwerkzugangspunkten (206A, 206B) umfasst:
mindestens einen Prozessor (502);
eine Netzwerkschnittstelle (514);
eine Eingabe-/Ausgabe-, EA-, Schnittstelle (512), die einen entsprechenden steckbaren Funkadapter (302) betriebsmäßig mit dem Netzwerkzugangspunkt (206A, 206B) verbindet; und
einen computerlesbaren Speicher (504), der eine Funkadapterkomponente (524) speichert, wobei die Funkadapterkomponente (524) Anweisungen enthält, die bei Ausführung durch den mindestens einen Prozessor bewirken, dass der Netzwerkzugangspunkt (206A, 206B) eines oder mehrere der Folgenden durchführt:
Einrichten eines Ende-zu-Ende-Datenpfads zwischen mindestens einem Patientenüberwachungs-Endgerät (112) und dem Patientenüberwachungs-Backend-System (110), wobei der Ende-zu-Ende-Datenpfad zumindest eine erste drahtlose Kommunikationsverbindung zwischen dem Endgerät (112) und einem entsprechenden steckbaren Funkadapter, der mit einem der Netzwerkzugangspunkte (206A, 206B) verbunden ist, sowie eine zweite Kommunikationsverbindung zwischen dem Netzwerkzugangspunkt (206A, 206B) und dem Backend-System (110) umfasst, wobei die erste Kommunikationsverbindung ein Netzwerkprotokoll verwendet, das dem ersten RF-Bereich entspricht, und die zweite Kommunikationsverbindung ein IEEE 802.3-Netzwerkprotokoll und/oder ein IEEE 802.11-Netzwerkprotokoll verwendet;
Empfangen von Daten von dem mindestens einen Endgerät (112) unter Verwendung des steckbaren Funkadapters (302) über die erste Kommunikationsverbindung des eingerichteten Ende-zu-Ende-Datenpfads; und
Übertragen der von dem mindestens einen Endgerät (112) empfangenen Daten unter Verwendung der Netzwerkschnittstelle (514) an das Backend-Gerät (110) über die zweite Kommunikationsverbindung des eingerichteten Ende-zu-Ende-Datenpfads.

5. Verfahren (800) zum Einrichten eines privaten Patientenüberwachungs-Netzwerksystems (300), wobei das Verfahren (800) umfasst:
Bereitstellen (802) einer Netzwerkinfrastruktur (108A, 108B), die eine Vielzahl von Netzwerkzugangspunkten (206A, 206B) umfasst;
Verbinden (804) einer Vielzahl steckbarer Funkadapter (302) mit der Vielzahl von Netzwerkzugangspunkten (206A, 206B), wobei jeder steckbare Funkadapter (302) umfasst:
ein erstes Kommunikationsfunkmodul (304), das so konfiguriert ist, dass es Daten innerhalb eines ersten Funkfrequenz-, RF-, Bereichs sendet und empfängt;
eine Funksteuerung (306), die betriebsmäßig mit dem ersten Kommunikationsfunkmodul (304) verbunden ist, wobei die Funksteuerung (306) so konfiguriert ist, dass sie Daten verarbeitet und über den steckbaren Funkadapter (302) weiterleitet; und
eine Geräteschnittstelle (308), die den steckbaren Funkadapter (302) betriebsmäßig mit einem entsprechenden Netzwerkzugangspunkt (206A, 206B) verbindet, wobei die Geräteschnittstelle (308) so konfiguriert ist, dass sie Daten zwischen dem steckbaren Funkadapter (302) und dem entsprechenden Netzwerkzugangspunkt (206A, 206B) weiterleitet; und
Einrichten (806) mindestens eines Ende-zu-Ende-Datenpfads zwischen mindestens einem Patientenüberwachungs-Endgerät (112) und einem Patientenüberwachungs-Backend-System (110), wobei der mindestens eine Ende-zu-Ende-Datenpfad zumindest eine erste Kommunikationsverbindung zwischen dem mindestens einen Patientenüberwachungs-Endgerät (112) und einem entsprechenden steckbaren Funkadapter, der mit einem der Vielzahl von Netzwerkzugangspunkten (206A, 206B) verbunden ist, sowie eine zweite Kommunikationsverbindung zwischen einem der Vielzahl von Netzwerkzugangspunkten (206A, 206B) und dem Patientenüberwachungs-Backend-System (110) umfasst, wobei die erste Kommunikationsverbindung ein Netzwerkprotokoll verwendet, das dem ersten RF-Bereich entspricht, und die zweite Kommunikationsverbindung entweder eine drahtgebundene Kommunikationsverbindung, eine drahtlose Kommunikationsverbindung oder eine Kombination aus diesen verwendet.

6. Verfahren (800) nach Anspruch 5, wobei die zweite Kommunikationsverbindung ein IEEE 802.3-Netzwerkprotokoll und/oder ein IEEE 802.11-Netzwerkprotokoll verwendet.

7. Verfahren (800) nach Anspruch 6, wobei der erste RF-Bereich innerhalb eines der folgenden Bereiche liegt: etwa 608 MHz bis etwa 614 MHz; etwa 1390 MHz bis etwa 1395 MHz; etwa 1395 MHz bis etwa 1400 MHz; etwa 1427 MHz bis etwa 1432 MHz; und/oder etwa 1432 MHz bis etwa 1435 MHz.

8. Verfahren nach einem der Ansprüche 6 oder 7, wobei eine Funkadapterkomponente (524) auf jedem Netzwerkzugangspunkt (206A, 206B) der Vielzahl von Netzwerkzugangspunkten (206A, 206B) ausgeführt wird, die eine Instanz einer Adapterverwaltungskomponente (530) innerhalb eines Netzwerkzugangspunkt-Host-Containers (600) auf dem Netzwerkzugangspunkt (206A, 206B) betreibt.

## Revendications

1. Système de réseau privé de surveillance de patient (300), le système de réseau privé de surveillance de patient (300) comprenant :
une infrastructure de réseau (108A, 108B) comprenant une pluralité de points d'accès au réseau (206A, 206B) ;
une pluralité d'adaptateurs radio enfichables (302), chaque adaptateur radio enfichable (302) étant connecté en fonctionnement à un point d'accès au réseau correspondant (206A, 206B), chaque adaptateur radio enfichable (302) comprenant :
au moins une première radio de communication (304, 312, 314) configurée pour envoyer et recevoir des données dans une première plage de fréquences radio, RF ;
un contrôleur radio (306) connecté en fonctionnement à au moins la première radio de communication (304, 312, 314), dans lequel le contrôleur radio (306) est configuré pour traiter et relayer des données par l'intermédiaire de l'adaptateur radio enfichable (302) ;
une interface de dispositif (308) connectant en fonctionnement l'adaptateur radio enfichable (302) au point d'accès au réseau correspondant (206A, 206B), l'interface de dispositif (308) étant configurée pour relayer des données entre l'adaptateur radio enfichable (302) et le point d'accès au réseau correspondant (206A, 206B) ;
un système dorsal de surveillance de patient (110) connecté en fonctionnement à l'infrastructure de réseau (108A, 108B) du système de réseau privé de surveillance de patient (300) ; et
un ou plusieurs dispositifs terminaux de surveillance de patient (112) en communication sans fil avec un ou plusieurs de la pluralité de points d'accès au réseau (206A, 206B), chaque dispositif terminal de surveillance de patient (112) étant configuré pour envoyer et recevoir des données en provenance du ou des points d'accès au réseau (206A, 206B), au moyen des adaptateurs radio enfichables connectés au ou aux points d'accès au réseau, dans au moins la première plage RF de l'adaptateur radio enfichable (302) qui est connecté à chacun du ou des points d'accès au réseau (206A, 206B) ; dans lequel le système de réseau privé de surveillance de patient est configuré pour établir au moins un trajet de données de bout en bout entre le ou les dispositifs terminaux de surveillance de patient du ou des dispositifs terminaux de surveillance de patient et le système dorsal de surveillance de patient, dans lequel le ou les trajets de données de bout en bout comprennent au moins une première liaison de communication entre le point d'accès de la pluralité de points d'accès au réseau et le dispositif terminal de surveillance de patient et l'adaptateur radio enfichable correspondant qui est connecté à l'un de la pluralité de points d'accès au réseau (206A, 206B), et une seconde liaison de communication entre le point d'accès de la pluralité de points d'accès au réseau et les systèmes dorsaux de surveillance de patient, la première liaison de communication utilisant un protocole de réseau correspondant à la première plage RF et la seconde liaison de communication utilisant soit une liaison de communication filaire, soit une liaison de communication sans fil, soit une combinaison de celles-ci.

2. Système privé de surveillance de patient (300) selon la revendication 1, dans lequel le contrôleur radio (306) de chaque adaptateur radio enfichable (302) est configuré pour relayer des données provenant d'au moins la première radio de communication (304, 312, 314), par l'intermédiaire de l'interface de dispositif (308) de l'adaptateur radio enfichable (302), et vers le point d'accès au réseau correspondant (206A, 206B) en tant que données série.

3. Système privé de surveillance de patient (300) selon l'une quelconque des revendications 1 et 2, dans lequel chaque adaptateur radio enfichable (302, 302B) comprend en outre :
une deuxième radio de communication (304, 312, 314) connectée en fonctionnement au contrôleur radio (306) et configurée pour envoyer et recevoir des données dans une deuxième plage RF, la deuxième plage RF étant différente de la première plage RF ; et
une troisième radio de communication (304, 312, 314) connectée en fonctionnement au contrôleur radio (306) et configurée pour envoyer et recevoir des données dans une troisième plage RF, la troisième plage RF étant différente des première et deuxième plages RF.

4. Système privé de surveillance de patient (300) selon l'une quelconque des revendications 1 à 3, dans lequel chaque point d'accès au réseau (206A, 206B) de la pluralité de points d'accès au réseau (206A, 206B) comprend :
au moins un processeur (502) ;
une interface réseau (514) ;
une interface d'entrée/sortie, E/S (512), connectant en fonctionnement un adaptateur radio enfichable correspondant (302) au point d'accès au réseau (206A, 206B) ; et
une mémoire lisible par ordinateur (504) stockant un composant adaptateur radio (524), le composant adaptateur radio (524) comprenant des instructions qui, lorsqu'elles sont exécutées par le ou les processeurs, amènent le point d'accès au réseau (206A, 206B) à exécuter une ou plusieurs des actions suivantes :
l'établissement d'un trajet de données de bout en bout entre au moins un dispositif terminal de surveillance de patient (112) et le système dorsal de surveillance de patient (110), dans lequel le trajet de données de bout en bout comprend au moins une première liaison de communication sans fil entre le dispositif terminal (112) et un adaptateur radio enfichable correspondant qui est connecté à l'un des points d'accès au réseau (206A, 206B), et une seconde liaison de communication entre le point d'accès au réseau (206A, 206B) et le système dorsal (110), la première liaison de communication utilisant un protocole de réseau correspondant à la première plage RF et la seconde liaison de communication utilisant au moins l'un d'un protocole de réseau IEEE 802.3 et d'un protocole de réseau IEEE 802.11 ;
la réception, par l'intermédiaire de la première liaison de communication du trajet de données de bout en bout établi, de données provenant du ou des dispositifs terminaux (112) au moyen de l'adaptateur radio enfichable (302) ; et
la transmission des données reçues depuis le ou les dispositifs terminaux (112), au moyen de l'interface réseau (514), au dispositif dorsal (110) par l'intermédiaire de la seconde liaison de communication du trajet de données de bout en bout établi.

5. Procédé (800) d'établissement d'un système réseau privé de surveillance de patient (300), le procédé (800) comprenant :
la fourniture (802) d'une infrastructure de réseau (108A, 108B) comprenant une pluralité de points d'accès au réseau (206A, 206B) ;
la connexion (804) d'une pluralité d'adaptateurs radio enfichables (302) à la pluralité de points d'accès au réseau (206A, 206B), chaque adaptateur radio enfichable (302) comprenant :
une première radio de communication (304) configurée pour envoyer et recevoir des données dans une première plage de fréquences radio, RF ;
un contrôleur radio (306) connecté en fonctionnement à la première radio de communication (304), dans lequel le contrôleur radio (306) est configuré pour traiter et relayer des données par l'intermédiaire de l'adaptateur radio enfichable (302) ; et
une interface de dispositif (308) connectant en fonctionnement l'adaptateur radio enfichable (302) à un point d'accès au réseau correspondant (206A, 206B), l'interface de dispositif (308) étant configurée pour relayer des données entre l'adaptateur radio enfichable (302) et le point d'accès au réseau correspondant (206A, 206B) ; et
l'établissement (806) d'au moins un trajet de données de bout en bout entre au moins un dispositif terminal de surveillance de patient (112) et un système dorsal de surveillance de patient (110), le ou les trajets de données de bout en bout comprenant au moins une première liaison de communication entre le ou les dispositifs terminaux de surveillance de patient (112) et un adaptateur radio enfichable correspondant connecté à l'un de la pluralité de points d'accès au réseau (206A, 206B), et une seconde liaison de communication entre l'un de la pluralité de points d'accès au réseau (206A, 206B) et le système dorsal de surveillance de patient (110), la première liaison de communication utilisant un protocole de réseau correspondant à la première plage RF et la seconde liaison de communication utilisant soit une liaison de communication filaire, soit une liaison de communication sans fil, soit une combinaison de celles-ci.

6. Procédé (800) selon la revendication 5, dans lequel la seconde liaison de communication utilise au moins l'un d'un protocole de réseau IEEE 802.3 et d'un protocole de réseau IEEE 802.11.

7. Procédé (800) selon la revendication 6, dans lequel la première plage RF est dans l'une des plages suivantes : environ 608 MHz à environ 614 MHz ; environ 1390 MHz à environ 1395 MHz ; environ 1395 MHz à environ 1400 MHz ; environ 1427 MHz à environ 1432 MHz ; et/ou environ 1432 MHz à environ 1435 MHz.

8. Procédé selon l'une quelconque des revendications 6 ou 7, dans lequel un composant adaptateur radio (524) est exécuté sur chaque point d'accès au réseau (206A, 206B) de la pluralité de points d'accès au réseau (206A, 206B) qui actionne une instance de composant de gestion d'adaptateur (530) dans un conteneur hôte de point d'accès au réseau (600) sur le point d'accès au réseau (206A, 206B).
